# EUROPEAN PATENT SPECIFICATION

(11) **EP 2 361 607 B1**
(45) Date of publication and mention of the grant of the patent: **25.03.2015**
(21) Application number: 10154002.9
(22) Date of filing: 18.02.2010
(51) Int. Cl.: A61K 8/81, A61Q 9/00, B26B 21/44, A61K 8/90, A61K 8/02, A61Q 9/04

(54) **Hair removal device comprising moisturizing compositions**
Haarentfernungsvorrichtung mit Feuchtigkeit spendenden Zusammensetzungen
Dispositif d'épilation comprenant des compositions humectantes

(43) Date of publication of application: 31.08.2011
(73) Proprietor: The Gillette Company, Boston, MA 02127 (US)
(72) Inventor: Stephens, Alison, Fiona, Maidenhead, Berkshire SL6 9LA (GB); Brooks, Alan, Woking, Surrey GU21 8UQ (GB)
(74) Representative: Kohol, Sonia

(56) References cited:
- WO-A1-92/05929
- WO-A1-2007/056509
- WO-A2-2007/139974
- GB-A- 2 423 494
- US-A1- 2004 136 944
- US-A1- 2008 209 734

## Description

### FIELD OF THE INVENTION

The present invention concerns the provision of a hair removal device comprising a solid moisturizing composition.

### BACKGROUND OF THE INVENTION

Hair removal devices incorporating a chemical composition are known and shall be referred to herein as devices comprising an "onboard" composition. Reference can be made to WO 07/056509 which teaches the inclusion of an onboard soap composition in a wet shaving razor. It is also known to provide a wet shaving razor incorporating an onboard skin-engaging composition comprising large quantities of hydrophilic polymers, such as polyethylene oxide, to lubricate the skin. Reference is made, by way of example, to WO 97/02116 and WO 97/02117.

The patent applications referred to above relate to the provision of various advantages, such as additional lathering and soap-related benefits, or improved lubrication in the case of polyethylene oxide. It would alternatively or additionally be advantageous to be able to provide a skin moisturizing benefit via an onboard chemistry, especially to male users who may be less motivated to use skin moisturizers than females. The provision of a moisturizing benefit from an onboard chemistry may, however, have a number of difficulties associated with it.

Skin moisturization may be achieved in several different ways, but one important formulational route to achieving skin moisturization is to include materials which bind water, such as polyols. However, polyols derive their water-binding abilities in part from their significant hydrophilicity, which may render them unsuited for use in any context involving water, such as is the case during wet shaving - they may be washed away during the initial stages of a shave. An alternative approach might be to use occlusive, hydrophobic materials which cover the skin and therefore act to retain water already present in the skin. These materials are occlusive hydrophobic emollients which are less likely to be washed away during use in a highly aqueous environment. WO 06/108522, discloses the use of small amounts of hydrophobic emollients in an onboard chemistry. GB 2423494, WO 92/05929, US 2008/0209734 and WO 2007/056509 also suggest moisturizing through onboard chemistry. However, formulations comprising significant proportions of hydrophobic emollients generally have mechanical and/or rheological properties which render them unsuitable for use as an onboard chemistry. For example, many hydrophobic emollients are liquids or low viscosity gels which flow easily and/or whose integrity may become substantially impaired by contact with hair, especially stiff male beard hair. One possible result, is that the emollient formulation may be mostly or entirely delivered at the start of the first use, over-delivering for that first use and leaving little or nothing for future uses of the hair removal device. Other hydrophobic emollients, such as some waxes, may not confer sufficient flexibility to an emollient composition. A further perceived problem associated with compositions comprising significant proportions of hydrophobic emollients is that they may increase drag across the skin, due to their affinity with the hydrophobic skin surface. An increase in friction is perceived as being a problem, because it is believed to result in a less pleasant hair removal experience.

### SUMMARY OF THE INVENTION

According to a first aspect of the invention, a hair removal device is provided comprising a solid moisturizing composition, the solid moisturizing composition comprising:
a. A linear ABA block copolymer, as defined in claim 1;
b. At least 50% moisturizing hydrophobic phase by weight of the solid moisturizing composition.

According to a second aspect of the invention, the use of a hair removal device according to the first aspect to remove hair and moisturize the skin is provided.

### DETAILED DESCRIPTION OF THE INVENTION

The "moisturizing hydrophobic phase", as herein defined, does not comprise and is separate from the linear ABA block copolymer.

As used herein, the term "solid" when used in relation to the moisturizing composition refers to compositions which are solid at 25°C and includes compositions which have a Chatillon Hardness at 25°C of 0.50 - 3.25kg, preferably 0.75 - 3.00kg, more preferably 1.00 - 2.50kg, measured according to the protocol provided hereinbelow.

The present applicants have established that a formulation rich in hydrophobic materials, but also comprising a linear ABA tri-block polymer has the requisite hardness to be successfully manufactured and used as an onboard chemistry in a hair removal device. Furthermore, the composition is erodable in a appropriate fashion, resulting in a formulation which wears down at a rate which may be regulated to provide hydrophobic emollient delivery during a plurality of separate hair-removal processes. Surprisingly, the present applicants have also established that the hydrophobic materials, while increasing drag, are actually appreciated by consumers, since they may assist in tensioning the skin, which is known to assist in the hair removal process.

The hair removal device according to the invention may be any hair removal device, such as, but not limited to, a razor, an epilator or a hair removal device comprising a light source to degrade or destroy the hair and/or hair root. If the hair removal device comprises a light source, then the light source may generate coherent (laser) light or incoherent light and may be adapted to generate light continuously or in a discontinuous (pulsed) fashion.

Preferably, the hair removal device is a razor. In the case of a razor, then the solid moisturizing composition may advantageously be provided on the razor cartridge, is preferably configured as a strip located before and/or after the blade(s) in the direction of cutting and may even be configured as a ring entirely surrounding the blades. WO 97/02116, referred to above, illustrates locations in which such a strip may be placed. Advantageously, the solid moisturizing composition is configured as a strip disposed after the cutting blade(s) in the cutting direction and, in this case, a lubricating strip comprising polyalkylene glycol is preferably additionally disposed before the cutting blades in the cutting direction.

According to the invention, the solid moisturizing composition comprises a linear ABA block copolymer. Preferably, the solid moisturizing composition comprises from 2% to 15% and more preferably from 3% to 12% linear ABA block copolymer by weight of the solid moisturizing composition. The linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof. More advantageously, the linear ABA block copolymer preferably comprises styrene-ethylenebutylene-styrene (S-EB-S) block copolymer.

More advantageously still, the weight ratio of styrene to butadiene in the S-EB-S is in the range 20:80 to 40:60 and preferably around 30:70. Particularly useful commercially available ABA block copolymers include Versagel^{™} materials available from Penreco and the Kraton^{™} G series, especially G-6150, G-1651, G-1652 and 1654.

According to the invention, the solid moisturizing composition comprises at least 50% moisturizing hydrophobic phase by weight of the solid moisturizing composition. Preferably, the solid moisturizing composition comprises from 60% to 95% and more preferably from 70% to 90% moisturizing hydrophobic phase by weight of the solid moisturising composition.

The moisturizing hydrophobic phase may comprise emollient which is liquid, semi-solid and/or solid at room temperature, which emollient may comprise one or more hydrocarbons, fatty acids, fatty alcohols, esters of a C₁₂ - C₃₀ alcohol, triglycerides, fats, butters, waxes, lipophilic skin active agents or mixtures thereof.

Advantageously, if solids or semi-solids are present, then the moisturizing hydrophobic phase comprises less than 20% and preferably less than 5% by weight of the solid moisturizing composition of materials, and more preferably no materials at all, having a melting point of more than 100°C. This is because excessive quantities of such materials may render the composition inflexible and therefore liable to crack during manufacture and/or use.

Liquid, semi-solid, or solid hydrocarbon emollients which may be comprised within the inventive composition include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD^{™} from Croda) and Polydecene (such as Puresyn 2^{™} from Exxon Mobil).

Liquid, semi-solid, or solid fatty alcohol or fatty acid emollients which may be comprised within the inventive composition include saturated and unsaturated higher alcohols, especially C₁₂ - C₃₀ fatty alcohols and fatty acids, especially lauric, myristic, palmitic, stearic , arachidic or behenic.

Liquid, semi-solid, or solid ester emollients which may be comprised within the inventive composition include esters of a C₁₂―C₃₀ alcohol and mixtures thereof, especially isopropyl myristate, isopropyl isostearate and mixtures thereof.

Liquid, semi-solid, or solid triglyceride emollients which may be comprised within the inventive composition include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol).

Liquid, semi-solid, or solid fat and butter emollients which may be comprised within the inventive composition include coconut butter, shea butter and mixtures thereof.

Liquid, semi-solid, or solid wax emollients which may be comprised within the inventive composition include paraffin wax, microcrystalline wax, candellila, ozokerite and mixtures thereof. Preferably the emollient comprises paraffin wax. Advantageously, moisturizing hydrophobic phase comprises some wax because waxes may bestow further improved hardness and erodability to the solid moisturising composition, although, as discussed above, the presence of too much wax may render the composition inflexible and therefore liable to crack during manufacture and/or use. Preferably, the solid moisturising composition comprises from 2% to 20% and more preferably from 3% to 15% wax by weight of the solid moisturising composition.

Liquid, semi-solid, or solid lipophilic skin active agent emollients which may be comprised within the inventive composition include oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The solid moisturising composition may comprise one or more additional components which bestow a suitable melt viscosity to the composition, such as oil phase gellants, to facilitate improved processing, provided that the additional component(s) do not significantly reduce the hardness or erodability of the solid moisturising composition. Examples of such components are trihydroxystearin, which is commercially available as Thixcin R™ (manufactured by Elementis Specialities), ethylene vinyl acetate (EVA) and mixtures thereof.

A solid moisturising composition comprised within the hair removal device of the invention may be manufactured by heating the elements of the moisturizing hydrophobic phase to a suitable temperature to melt them, typically approximately 130°C, after which the linear ABA tri-block polymer is added and mixed well until the ABA tri-block polymer has dissolved. The mixture is then cooled, typically to approximately 90°C, after which any additional ingredients may be added. In a final step, the mixture is poured into suitable containers or moulds and allowed to cool to room temperature.

Once the mixture has set, it may be affixed to a hair removal device in any appropriate fashion. One such approach would involve mechanically fitting the composition onto the hair removal device. Alternatively, the composition may be directly or indirectly adhered to the hair removal device by means of an adhesive composition. One method of indirect adherence involves casting the solid moisturizing composition onto a sheet of an appropriate substrate, such as an acetate sheet, which sheet is then adhered to the hair removal device, for example mechanically or via an adhesive.

### Chatillon Hardness test

Equipment: Chatillon TCD 200 equipped with a digital force gauge

### Sample preparation

1. Fully melt and cast lipid into 60ml weigh boat (70mm X 70mm X 24mm)
2. Store lipid at 25°C overnight to equibrilate
3. Carefully remove lipid from weigh boat prior to hardness testing

### Machine Preparation

A)
   1. Prepare Chatillon TCD 200 and digital force gauge according to manufacturers instructions.
   2. Set the ramp speed to 47 mm / min
B) Measuring the hardness value at 25°C:
   1. The pointed geometry should be attached to the shaft of ramp for this test method.
   2. Place the lipid sample as prepared above and on its side onto the metal base plate directly below the centre of the shaft of the ramp. The mid-point of the lipid should be in line with the centre of the shaft of the ramp.
   3. With the lipid in place below the flat plate the speed set at 47 mm/min and the digital force gauge set at "C Peak" as above, depress the "Down" button on the Chatillon TCD200.
   4. Stop the Chatillon TCD200 just as the probe touches the surface of the lipid and set the distance counter to zero.
   5. Reset the force gauge so that it reads zero
   6. Depress the "Down" button on the Chatillon TCD200 untill the distance counter reads 13mm, record C Peak reading.

### Examples

The following examples disclose solid moisturizing compositions, which were incorporated into razors as a strip disposed after the blade in the cutting direction. In use, they were observed to remove hair and deposit the solid moisturizing composition onto the skin from which hair had been removed.

### Example 1

| **Trade Name** | **INCI Name** | **% w/w** |
|---|---|---|
| White soft paraffin | Petrolatum | 44.0 |
| Mineral oil | Paraffinum Liquidum | 44.0 |
| Kraton G1650E | Hydrogenated Styrene/Butadiene copolymer | 5.0 |
| Thixcin R | Trihydroxystearin | 2.0 |
| Paraffin Wax SP206 | Paraffin | 5.0 |

The composition of Example 1 is manufactured by heating the hydrocarbons and waxes to 130°C, then adding the linear ABA tri-block polymer (Kraton G1650E) and mixing well until polymer has fully dissolved. The mixture is then cooled to 90°C and the Thixcin added, after which the mixture is poured into a mould and allowed to cool to room temperature.

The Chatillon Hardness of the formulation of Example 1 is 1.7.

### Example 2

| **Trade Name** | **INCI Name** | **% w/w** |
|---|---|---|
| Fractionated coconut oil | Caprylic/Capric Triglyceride | 85.0 |
| Paraffin Wax SP206 | Paraffin | 7.5 |
| Kraton GRP6935 | Hydrogenated Styrene/Butadiene copolymer | 5.0 |
| Synthetic Beeswax | Arachidyl Behenate | 2.5 |

The composition of Example 2 is manufactured by heating the hydrocarbons and waxes to 130°C, then adding the linear ABA tri-block polymer (Kraton GRP6935) and mixing well until polymer has fully dissolved. The mixture is then cooled to 90°C, after which the mixture is poured into a mould and allowed to cool to room temperature.

The Chatillon Hardness of the formulation of Example 2 is 0.6.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A hair removal device comprising a solid moisturizing composition, the solid moisturizing composition comprising:
a. A linear ABA block copolymer; wherein the linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof,
b. At least 50% moisturizing hydrophobic phase by weight of the solid moisturizing composition.

2. The hair removal device of claim 1, wherein the solid moisturizing composition comprises from 2% to 15%, preferably from 3% to 12% linear ABA block copolymer by weight of the solid moisturizing composition.

3. The hair removal device of claim 1, wherein the linear ABA block copolymer comprises styrene-ethylenebutylene-styrene (S-EB-S) block copolymer.

4. The hair removal device according to any preceding claim, wherein the moisturizing. hydrophobic phase comprises emollient which is liquid, semi-solid or solid at room temperature.

5. The hair removal device according to any preceding claim, wherein the solid moisturizing composition comprises from 60% to 95%, preferably from 70% to 90% moisturizing hydrophobic phase by weight of the solid moisturising composition.

6. The hair removal device according to any preceding claim, wherein the moisturizing hydrophobic phase comprises hydrocarbons, fatty acids, fatty alcohols, esters of a C₁₂-C₃₀ alcohol, triglycerides, fats, butters, waxes or mixtures thereof.

7. The hair removal device according to any preceding claim, wherein the solid moisturising composition comprises from 2% to 20% and more preferably from 3% to 15% wax by weight of the solid moisturising composition.

8. The hair removal device of any preceding claim, configured as a razor.

9. The razor according to claim 7, wherein the solid moisturizing composition is configured as a strip disposed on one or both sides of the cutting blade(s).

10. The razor according to claim 8 or 9 wherein the solid moisturizing composition is configured as a strip disposed after the cutting blade(s) in the cutting direction and wherein a lubricating strip comprising polyalkylene glycol is disposed before the cutting blades in the cutting direction.

11. The hair removal device of any of claims 1 to 7, wherein the hair removal device comprises a light source, preferably a laser light source, to degrade or destroy the hair or hair root.

12. Use of a hair removal device according to any preceding claim to remove hair and moisturize the skin.

## Patentansprüche

1. Haarentfernungsvorrichtung, umfassend eine feste feuchtigkeitsspendende Zusammensetzung, wobei die feste feuchtigkeitsspendende Zusammensetzung Folgendes umfasst:
a. ein lineares ABA-Blockcopolymer; wobei das lineare ABA-Blockcopolymer ein Styrol-Butadien-Styrol (SBS)-Blockcopolymer, ein Styrol-Isopren-Styrol (SIS)-Blockcopolymer, ein Styrol-Etlrylenbutylen-Styrol (S-EB-S)-Blockcopolymer oder Mischungen davon umfasst,
b. mindestens 50 Gew.-% feuchtigkeitsspendende hydrophobe Phase bezogen auf die feste feuchtigkeitsspendende Zusammensetzung.

2. Haarentfernungsvorrichtung nach Anspruch 1, wobei die feste feuchtigkeitsspendende Zusammensetzung von 2 Gew.-% bis 15 Gew.-%, vorzugsweise von 3 Gew.-% bis 12 Gew.-%, lineares ABA-Blockcopolymer bezogen auf die feste feuchtigkeitsspendende Zusammensetzung umfasst.

3. Haarentfernungsvorrichtung nach Anspruch 1, wobei das lineare ABA-Blockcopolymer Styrol-Ethylenbutylen-Styrol (S-EB-S)-Blockcopolymer umfasst.

4. Haarentfernungsvorrichtung nach einem der vorstehenden Ansprüche, wobei die feuchtigkeitsspendende hydrophobe Phase einen Weichmacher umfasst, der bei Raumtemperatur flüssig, halbfest oder fest ist.

5. Haarentfernungsvorrichtung nach einem der vorstehenden Ansprüche, wobei die feste feuchtigkeitsspendende Zusammensetzung von 60 Gew.-% bis 95 Gew.-%, vorzugsweise von 70 Gew.-% bis 90 Gew.-%, feuchtigkeitsspendende hydrophobe Phase bezogen auf die feste feuchtigkeitsspendende Zusammensetzung umfasst.

6. Haarentfernungsvorrichtung nach einem der vorstehenden Ansprüche, wobei die feuchtigkeitsspendende hydrophobe Phase Kohlenwasserstoffe, Fettsäuren, Fettalkohole, Ester eines C₁₂-C₃₀-Alkohols, Triglyceride, Fette, Butter, Paraffine oder Mischungen davon umfasst.

7. Haarentfernungsvorrichtung nach einem der vorstehenden Ansprüche, wobei die feste feuchtigkeitsspendende Zusammensetzung von 2 Gew.-% bis 20 Gew.-% und weiter bevorzugt von 3 Gew.-% bis 15 Gew.-%, Paraffin bezogen auf die feste feuchtigkeitsspendende Zusammensetzung umfasst.

8. Haarentfernungsvorrichtung nach einem der vorstehenden Ansprüche, die als Rasierer ausgeführt ist.

9. Rasierer nach Anspruch 7, wobei die feste feuchtigkeitsspendende Zusammensetzung als ein Streifen ausgeführt ist, der auf einer Seite oder auf beiden Seiten des/der Schneidmesser(s) angeordnet ist.

10. Rasierer nach Anspruch 8 oder 9, wobei die feste feuchtigkeitsspendende Zusammensetzung als ein Streifen ausgeführt ist, der nach dem/den Schneidmesser(n) in der Schneidrichtung angeordnet ist, und wobei ein Gleitstreifen, der Polyalkylenglykol umfasst, vor den Schneidmessern in der Schneidrichtung angeordnet ist.

11. Haarentfernungsvorrichtung nach einem der Ansprüche 1 bis 7, wobei die Haarentfernungsvorrichtung eine Lichtquelle, vorzugsweise eine Laserlichtquelle, umfasst, um das Haar oder die Haarwurzel abzubauen oder zu zerstören.

12. Verwendung einer Haarentfernungsvorrichtung nach einem der vorstehenden Ansprüche, zum Entfernen von Haaren und um der Haut Feuchtigkeit zu spenden.

## Revendications

1. Dispositif d'épilation comprenant une composition hydratante solide, la composition hydratante solide comprenant :
a. Un copolymère séquencé ABA linéaire ; dans lequel le copolymère séquencé ABA linéaire comprend un copolymère séquencé styrène-butadiène-styrène (SBS), un copolymère séquencé styrène-isoprène-styrène (SIS), un copolymère séquencé styrène-éthylène-butylène-styrène (S-EB-S), ou des mélanges de ceux-ci,
b. Au moins 50 % d'une phase hydrophobe hydratante en poids de la composition hydratante solide.

2. Dispositif d'épilation selon la revendication 1, dans lequel la composition hydratante solide comprend de 2 % à 15 %, de préférence de 3 % et à 12 % de copolymère séquencé ABA linéaire en poids de la composition hydratante solide.

3. Dispositif d'épilation selon la revendication 1, dans lequel le copolymère séquencé ABA linéaire comprend un copolymère séquencé styrène-éthylène-butylène-styrène (S-EB-S).

4. Dispositif d'épilation selon l'une quelconque des revendications précédentes, dans lequel la phase hydrophobe hydratante comprend un émollient qui est liquide, semi-solide ou solide à température ambiante.

5. Dispositif d'épilation selon l'une quelconque des revendications précédentes, dans lequel la composition hydratante solide comprend de 60 % à 95 %, de préférence de 70 % à 90 % d'une phase hydrophobe hydratante en poids de la composition hydratante solide.

6. Dispositif d'épilation selon l'une quelconque des revendications précédentes, dans lequel la phase hydrophobe hydratante comprend des hydrocarbures, des acides gras, des alcools gras, des esters d'un alcool en C 12 à C30, des triglycérides, des graisses, des beurres, des cires ou des mélanges de ceux-ci.

7. Dispositif d'épilation selon l'une quelconque des revendications précédentes, dans lequel la composition hydratante solide comprend de 2 % à 20 % et plus préférentiellement de 3 % à 15 % de cire en poids de la composition hydratante solide.

8. Dispositif d'épilation selon l'une quelconque des revendications précédentes, configuré sous forme de rasoir.

9. Rasoir selon la revendication 7, dans lequel la composition hydratante solide est configurée sous forme de bande disposée sur un ou sur les deux côtés de la lame ou des lames de coupe.

10. Rasoir selon la revendication 8 ou 9, dans lequel la composition hydratante solide est configurée sous forme de bande disposée après la lame ou les lames de coupe dans la direction de coupe et dans lequel une bande de lubrification comprenant un polyalkylèneglycol est disposée devant les lames de coupe dans la direction de coupe.

11. Dispositif d'épilation selon l'une quelconque des revendications 1 à 7, dans lequel le dispositif d'épilation comprend une source de lumière, de préférence une source de lumière laser, pour dégrader ou détruire le poil ou la racine du poil.

12. Utilisation d'un dispositif d'épilation selon l'une quelconque des revendications précédentes pour éliminer les poils et hydrater la peau.
